# EUROPEAN PATENT APPLICATION

(11) **EP 2 434 009 A1**
(43) Date of publication of application: **28.03.2012**
(21) Application number: 10184985.9
(22) Date of filing: 19.05.2005
(51) Int. Cl.: C12N 9/14

(54) **Modulation of WRN-mediated telomere-initiated cell signaling**

(30) Priority: 19.05.2004 US 572625 P
(62) Divisional of application: 05750059.7
(71) Applicant: Trustees of Boston University, Boston, MA 02215 (US)
(72) Inventor: Eller, Mark S., Boston, MA 02118 (US); Gilchrest, Barbara, Boston, MA 02109 (US)
(74) Representative: den Hartog, Jeroen H.J.

(57) **Abstract**

The invention relates to a composition comprising an activator or inhibitor of WRN for use as a medicament, wherein the use comprises the treatment of cancer, the inducement of apoptosis, inducing cellular senescence treatment of promoting tanning, promoting cellular differentiation or promoting immunosuppression. The activator or inhibitor can be an oligonucleotide activator or inhibitor of WRN with at least 33% nucleotide sequence identity with (TTAGGG)ₙ and at least the first x 3'-nucleotide linkages are hydrolyzable by a 3' to 5' nuclease, wherein n=1 to 20, and wherein x is from about 0 to about 10.

## Description

### BACKGROUND OF THE INVENTION

### 1. Field of the Invention

The present invention relates to the regulation of signaling pathways. More specifically, the present invention relates to the regulation of telomere-initiated senescence, apoptosis, tanning and other DNA damage responses.

### 2. Description of Related Art

The frequency of cancer in humans has increased in the developed world as the population has aged. For some types of cancers and stages of disease at diagnosis, morbidity and mortality rates have not improved significantly in recent years in spite of extensive research. During the progression of cancer, tumor cells become more and more independent of negative regulatory controls, including resistance to senescence and apoptosis, the important aspects of how the interaction of normal cells with their tissue-specific environment is regulated.

Cellular senescence has been suggested to be an important defense against cancer. Extensive evidence implicates progressive telomere shortening (caused by an inability to replicate the 3' ends of chromosomes) or some other form of telomere dysfunction in senescence. In germline cells and most cancer cells, immortality is associated with maintenance of telomere length by telomerase, an enzyme complex that adds TTAGGG repeats to the 3' terminus of the chromosome ends. Telomeres, tandem repeats of TTAGGG, end in a loop structure with a 3' single-stranded overhang of approximately 150-300 bases tucked within the proximal telomere duplex DNA and stabilized by telomeric repeat binding factors (TRFs), particularly TRF2. Ectopic expression of a dominant-negative form of TRF2 (TRF2^{DN}) disrupts telomere loop structure, exposes the 3' overhang and causes DNA damage responses, followed by senescence in primary fibroblasts, fibrosarcoma cells, and several other malignant cell types.

Senescence can also be precipitated acutely by extensive DNA damage or the overexpression of certain oncogenes. Ectopic expression of the telomerase reverse transcriptase catalytic subunit (TERT), which enzymatically maintains or builds telomere length, can bypass senescence with subsequent immortalization of some human cell types, strongly suggesting a telomere-dependent mechanism of replicative senescence. Moreover, malignant cells commonly express TERT and/or contain mutations that allow the cell to bypass the senescent response and to proliferate indefinitely despite often having shorter telomeres than normal senescent cells. However, some tumor cells undergo senescence in response to various anticancer agents, indicating that acquisition of immortality does not necessarily imply a loss of this basic cellular response to DNA damage.

Senescence in human cells is largely dependent on the p53 and pRb pathways. The tumor suppressor p53 plays a key role in cellular stress response mechanisms by converting a variety of different stimuli, for example, DNA damage, deregulation of transcription or replication, oncogene transformation, and deregulation of microtubules caused by some chemotherapeutic drugs, into cell growth arrest or apoptosis. When activated, p53 causes cell growth arrest or a programmed, suicidal cell death (apoptosis), which in turn acts as an important control mechanism for genomic stability. In particular, p53 controls genomic stability by eliminating genetically damaged cells from the cell population, and thus one of its major functions is to prevent tumor formation.

An intact tumor suppressor pRb pathway also contributes to preventing tumorigenesis. In pRb^{-/-} tumor cells that do not contain wild-type p53, introduction ofpRb induces senescence. Although cervical cancer cells frequently retain wild-type p53 and pRb genes, the HPV E6 and E7 proteins interfere with the p53 and pRb pathways, respectively. Ectopic expression of viral - E2 protein represses HPV E6 and E7 gene transcription and induces a rapid and prominent senescent response in cervical carcinoma cell lines, again affirming the important roles of p53 and pRb in cancer cell senescence.

Suppressing only the p53 or the pRb pathway is not sufficient for fibroblasts to bypass replicative senescence. Indeed, human fibroblasts either transfected with SV 40 T antigen or transduced with combinations of adenovirus E1A+E1B or HPV E6+E7, suppressing both the p53 and pRb pathways, have an extended life span and escape replicative senescence.

Double strand breaks in DNA are extremely cytotoxic to mammalian cells. The highly conserved MRN complex is involved in the repair of double strand breaks in eukaryotes. The MRN complex adheres to sites of double strand breaks immediately following their formation. The MRN complex also migrates to telomeres during the S-phase of the cell cycle associates with telomeric repeat binding factors (TRF).

The MRN complex consists of Mre11, Rad50 and NBS (p95). Mre11, as part of the Mre11/p95/Rad50 complex, associates with the telomere during S phase of the cell cycle. Mre11 is an exonuclease with preference for the 3' end of a DNA strand. The activity of Mre11 is believed to be dependent on interaction with Rad50, which is an ATPase. Nbs 1 is believed to be involved in the nuclear localization of the MRN complex, as well as its assembly at the site of a double strand break.

A protein mutated in Werner's Syndrome, the WRN protein, is known to interact with the MRN complex (Cheng *et al.,* 2004, Vol. 2004). Werner's Syndrome is an autosomal recessive disorder that is characterized by premature aging, increased malignancies and genomic instability. WRN is a nuclear protein that contains both helicase and 3' to 5' exonuclease domains (Oshima, J., 2002, Bioessays 22, 894-901). To date, all mutations identified in Werner's Syndrome are WRN truncations that eliminate the nuclear localization signal from the COOH end of the protein (Oshima, J., 2002). Therefore, it is believed that WRN mutations in Werner's s Syndrome generate a functional null phenotype by preventing the protein from reaching its site of action in the nucleus. Cells from Werner's Syndrome patients show increased levels of deletions and translocations, both baseline and after DNA damage, suggesting that the WRN protein participates in DNA repair, replication and recombination (Opresko et al., 2003, .. Carcinogenesis 24, 791-802). Werner's Syndrome cells also senesce prematurely compared to age-matched controls (Martin et al., 1970, Lab Invest 23, 86-92) and also demonstrate accelerated telomere shortening (Schulz et al., 1996, Hum Genet 97, 750-4)

In addition to interactivng with the MRN complex, WRN is known to interact with other proteins that participate in DNA damage responses and DNA repair/replication: DNA-PK/Ku (Karmakar et al., 2002, Nucleic Acids Res 30, 3583-91), p53 (Brosh et al., 2001, J Biol Chem 276, 35093-102), and the helicase mutated in the premature aging syndrome, Bloom's Syndrome, BLM (von Kobbe et al., 2002, J Biol Chem 277, 22035-44). Furthermore, WRN interacts with telomere repeat-binding factor 2, TRF2, and this interaction alters the specificity of the WRN exonuclease activity to facilitate 3' to 5' digestion of the telomeric DNA (Machwe et al., 2004, Oncogene 23, 149-56; Opresko et al., 2002, J Biol Chem 277, 41110-9). Together, these data demonstrate a critical role for WRN in DNA metabolism and telomere maintenance. However the precise role of WRN in these pathways is not understood.

Cancers are typically treated with highly toxic therapies, such as chemotherapy and radiation therapy, that comparably damage all proliferative cells whether normal or malignant. Side effects of such treatments include severe damage to the lymphoid system, hematopoietic system and intestinal epithelia, as well as hair loss. There continues to be a need for safer and more effective cancer therapies, especially for alternative therapies that would avoid some or all of these side effects by preferentially targeting malignant cells relative to normal but proliferative cells.

### SUMMARY OF THE INVENTION

The present invention relates to a cell-based method of screening for a modulator of WRN, comprising contacting a candidate modulator with a cell that expresses WRN under conditions in which the modulator is taken up by the cell, and measuring a property of the cell associated with activation of the DNA damage response pathway including, but not limited to, cellular proliferation, cellular viability, cellular morphology, SA-β-Gal activity, and phosphorylation of p53 or p95, phosphorylation of ATM, phosphorylation of H2AX, induction of S phase arrest or induction of apoptosis. A modulator may be identified by altering the property compared to a control. The candidate modulator may be an agent that specifically binds to WRN. WRN may be expressed as a fragment, homolog, analog or variant of WRN, which may have exonuclease activity.

The present invention also relates to an *in vitro* method of screening for an agent that specifically binds to WRN, comprising contacting a candidate agent with WRN, and determining whether the candidate agent specifically binds to WRN. WRN may be attached to a solid support. Alternatively, the candidate agent may be attached to a solid support.

The present invention also relates to an *in vitro* method of screening for a modulator of WRN comprising contacting a candidate modulator with WRN *in vitro* in the presence of a nucleic acid substrate for WRN, and measuring the hydrolysis of the substrate. A modulator may be identified by altering hydrolysis of the substrate nucleic acid compared to a control. The nucleic acid substrate may be an oligonucleotide with at least 33% nucleotide sequence identity with (TTAGGG)ₙ, wherein n=1 to 20. Alternatively, the nucleic acid substrate may be an oligonucleotide with at least 50% nucleotide sequence identity with (TTAGGG)ₙ, wherein n=1 to 20. The hydrolysis of the substrate nucleic acid may be measured by UV absorbance, gel analysis of labeled oligos, or recovery of non-precipitatable nucleotide bases.

The present invention also relates to a cell-based method of screening for a modulator of the DNA damage pathway, comprising contacting a candidate modulator with a cell that expresses WRN in the presence of an oligonucleotide under conditions in which the modulator is taken up by the cell, and measuring a property of the cell associated with activation of the DNA damage response pathway including, but not limited to, cellular proliferation, cellular viability, cellular morphology, senescence associated β-galactosidase (SA-β-Gal) activity and phosphorylation of p53 or p95; phosphorylation of ATM, phosphorylation of H2AX, induction of S phase arrest or induction of apoptosis. A modulator may be identified by altering the property compared to a control. The oligonucleotide may have at least 33% nucleotide sequence identity with (TTAGGG)ₙ, wherein n=1 to 20. The oligonucleotide may have at least 50% nucleotide sequence identity with (TTAGGG)ₙ, wherein n=1 to 20. WRN may be expressed as a fragment, homolog, analog or variant of WRN, which may have exonuclease activity.

The cell used in the cell-based screening methods described above may be a cancer cell. The cell used in the cell-based screening methods described may maintain telomeres by telomerase reverse transcriptase or the ALT pathway. The candidate modulators and agents described in the *in vitro* and cell-based screening methods above may be carbohydrates, monosaccharides, oligosaccharides, polysaccharides, amino acids, peptides, oligopeptides, polypeptides, proteins, nucleosides, nucleotides, oligonucleotides, polynucleotides, lipids, retinoids, steroids, glycopeptides, glycoproteins, proteoglycans, or small organic molecules.

The present invention also relates to the use of compositions comprising an activator of WRN. The activator may be used for treating cancer, inducing apoptosis, inducing cellular senescence, inhibiting promoting tanning, promoting cellular differentiation or promoting immunosuppression. The activator may be an oligonucleotide activator of WRN, which may have at least 33% nucleotide sequence identity with (TTAGGG)ₙ, wherein n=1 to 20. The activator may be an oligonucleotide activator of WRN, which may have at least 50% nucleotide sequence identity with (TTAGGG)ₙ, wherein n=1 to 20. From about one to about ten of the first 3'-nucleotide linkages may be hydrolyzable by a 3' to 5' nuclease.

The present invention also relates to the use of compositions comprising an inhibitor of WRN. The inhibitor may be used to inhibit apoptosis, inhibit cellular senescence, promote growth, inhibit tanning, inhibit cellular differentiation, or reduce cancer treatment side effects. The composition may be given in combination with chemotherapy or ionizing radiation. The inhibitor may be an oligonucleotide inhibitor of WRN, which may have at least 33% nucleotide sequence identity with (TTAGGG)ₙ, wherein n=1 to 20. The inhibitor may be an oligonucleotide inhibitor of WRN, which may have at least 50% nucleotide sequence identity with (TTAGGG)ₙ, wherein n=1 to 20. From about zero to about ten of the first 3'-nucleotide linkages may be hydrolyzable by a 3' to 5' nuclease.

The present invention also relates to a composition comprising an oligonucleotide with at least 33% nucleotide sequence identity with (TTAGGG)ₙ and with at least one nonhydrolyzable internucleotide linkage, wherein n=1 to 20. From one to about ten of the first 3'-nucleotide linkages may be hydrolyzable by a 3' to 5' nuclease, such as WRN. The oligonucleotide may have at least 33% nucleotide sequence identity with TTAGGG. The oligonucleotide may also have at least 50% nucleotide sequence identity with TTAGGG. The oligonucleotide may also have the sequence GTTAGGGTTAG. The nonhydrolyzable linkage may be a phosphorothioate. The oligonucleotide may be a PNA.

### BRIEF DESCRIPTION OF THE DRAWINGS

Figures 1A-1H show FACS analysis ofpropidium iodide stained Jurkat cells (immortalized T lymphocytes), treated with diluent (Figures 1A and 1E); 40 µM 11mer-1 pGTTAGGGTTAG (SEQ ID NO: 2) (Figures 1B and 1F); 40 µM 11mer-2 pCTAACCCTAAC (SEQ ID NO: 3) (Figures 1C and 11G); 40 µM 11mer-3 pGATCGATCGAT (SEQ ID NO: 4) (Figures 1D and 1H). Jurkat cells were treated with the stated reagents for 48 hours before analysis (Figures 1A-1D) or 72 hours (Figures 1E-1H).

Figures 2A-2F are profiles showing the results of fluorescence activated cell sorting, for the following additions to the cells: Figure 2A, diluent; Figure 2B, 0.4 µM 11mer-1; Figure 2C, 0.4 µM 11mer-1-S; Figure 2D, diluent; Figure 2E, 40 µM 1 lmer-1; Figure 2F, 40 µM 11mer-1-S.

Figures 3A-3G are profiles showing the results of fluorescence activated cell sorting, for the following additions to the cells: Figure 3A, diluent; Figure 3B, 10 µM 11mer-1; Figure 3C, 10 µM 11mer-1 and 1 µM 11mer-1-S; Figure 3D, 10 µM 11mer-1 and 5 µM 11mer-1-S; Figure 3E, 10 µM 11mer-1 and 10 µM 11mer-1-S; Figure 3F, 20µM 11mer-1-S; Figure 3G, 10 µM 11mer-1-S.

Figure 4 is a bar graph showing the melanin content (in pg/cell) of cells treated with diluent, pTpT or pTspT.

Figure 5 is a bar graph showing the melanin content (in pg/cell) of cells treated with diluent, 11mer-1 or 11mer-1-S.

Figure 6 is a bar graph showing the melanin content (in pg/cell) of cells that have been sham-treated (no irradiation, no oligonucleotides), or treated with ultraviolet light (UV), or unirradiated but given pTspT, or irradiated with UV and given pTspT.

Figure 7 is a diagram of oligonucleotides of nucleotide sequence SEQ ID NO: 2 which were synthesized with phosphorothioate linkages.

Figure 8 is a bar graph showing the results of testing the effects of phosphorothioate oligonucleotides 1, 2, 3 and 4 depicted in Figure 7 in causing senescence in cultures of normal neonatal human fibroblasts, indicated by the cells staining positive for β-galactosidase activity. Oligonucleotide "11-1" indicates fibroblast cultures treated with SEQ ID NO: 2 synthesized entirely with phosphodiester linkages. "Dil" indicates fibroblast cultures treated with diluent not containing oligonucleotide.

Figure 9 shows the ability of T-oligo to induce the phosphorylation of p53 and H2AX is reduced by knocked down WRN. Protein level of WRN on the day of T-oligo treatment is indicated at "0 Hrs". T-oligo (40 *µ*M, T) or diluent (D) were added and cells collected after 24 or 48 hours for western analysis. Control fibroblasts were either sham (IR,-) or irradiated with 10 Gy IR (IR, +) and collected after one hour. The western blot was probed with antibodies recognizing WRN, p53 phosphoserine 15 or phospho H2AX.

Figure 10 shows that T-oligo leads to phosphorylation of the ATM-related DNA-dependent protein kinase (DNA-PK) catalytic subunit (DNA-PK_{cs}). The positions of ATM and DNA-PK_{cs} are indicated. Sham and irradiated (10 Gy IR) fibroblasts were included as controls.

Figure 11 shows that serine 37 of p53 is phosphorylated in response to T-oligo treatment.

Figure 12 shows a potential mechanism for the WRN protein "sensing" exposed telomere overhangs and T-oligos, leading to activation of ATM and DNA-PK, leading to the phosphorylation of p53 as well as other substrates.

### DETAILED DESCRIPTION OF THE INVENTION

The present invention is based on the discovery that WRN is involved in DNA damage-like signaling that is initiated by hydrolysis of the 3' telomere overhang sequence. Hydrolysis of telomere sequences initiates signaling cascades important for protective cellular responses to DNA damage including but not limited to cell senescence, tanning and apoptosis. As shown here and in copending International Patent Application No. PCT/US2004/000819, which is incorporated by reference in its entirety, T-oligos blocked to nuclease digestion at the 3' end lose their ability to stimulate DNA damage responses. In view of WRN being a 3' to 5' nuclease involved in DNA replication and repair and telomere maintenance, WRN may be a nuclear "sensor" of T-oligos.

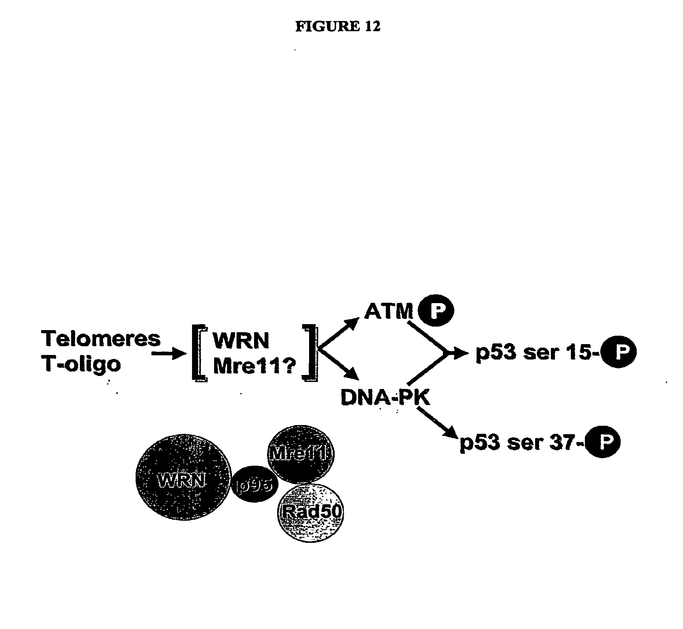

Not being bound by theory, we believe that DNA damage, such as UV irradiation, oxidative damage to DNA, or formation of carcinogen adducts to DNA, or age-associated telomere shortening destabilizes the telomere loop, exposing the 3' overhang sequence comprising repeats of TTAGGG. Telomere-associated proteins then attach to the overhang in a sequence-dependent manner and serve as an "anchor" for WRN, either by itself or as part of a complex. WRN then begins to hydrolyze the telomere overhang from the 3' end, which leads to a further signaling cascade that ultimately leads to the biologic endpoints of cell cycle arrest, gene induction, apoptosis and/or senescence. The data presented herein suggest that the WRN protein, either alone or as part of a complex, such as the Mre11/Rad50/p95(Nbs1) complex, "senses" and degrades T-oligos, leading to activation of the ATM and DNA-PK kinases and the subsequent phosphorylation of downstream substrates (Figure 12).

Based on the role of WRN in the proposed signaling pathway, activators of WRN are expected to activate the DNA damage response pathway regardless of the presence of DNA damage or telomere loop disruption. Similarly, inhibitors of WRN are expected to inhibit the signal transduction pathway, even in the presence of DNA damage or telomere loop disruption.

Before the present products, compositions and methods are disclosed and described, it is to be understood that the terminology used herein is for the purpose of describing particular embodiments only and is not intended to be limiting. It must be noted that, as used in the specification and the appended claims, the singular forms "a," "an" and "the" include plural referents unless the context clearly dictates otherwise.

Throughout this application, where patents or publications are referenced, the disclosures of these publications in their entireties are hereby incorporated by reference into this application in order to more fully describe the state of the art to which this invention pertains.

### 1. Definitions

As used herein, the term "activator" means anything that activates a protein or increases the activity of a protein.

As used herein, the term "administer" when used to describe the dosage of a modulator means a single dose or multiple doses of the agent.

As used herein, the term "analog", when used in the context of a peptide or polypeptide, means a peptide or polypeptide comprising one or more non-standard amino acids or other structural variations from the conventional set of amino acids, and preferably retains at least one biological activity; and, when used in the context of an oligonucleotide, means an oligonucleotide comprising one or more internucleotide linkages other than phosphodiester internucleotide linkages, and preferably retains at least one biological activity.

As used herein, the term "antibody" means an antibody of classes IgG, IgM, IgA, IgD or IgE, or fragments or derivatives thereof, including Fab, F(ab')₂, Fd, and single chain antibodies, diabodies, bispecific antibodies, bifunctional antibodies and derivatives thereof. The antibody may be a monoclonal antibody, polyclonal antibody, affinity purified antibody, or mixtures thereof which exhibits sufficient binding specificity to a desired epitope or a sequence derived therefrom. The antibody may also be a chimeric antibody. The antibody may be derivatized by the attachment of one or more chemical, peptide, or polypeptide moieties known in the art. The antibody may be conjugated with a chemical moiety.

As used herein, "apoptosis" refers to a form of cell death that includes, but is not limited to, progressive contraction of cell volume with the preservation of the integrity of cytoplasmic organelles; condensation of chromatin (i.e., nuclear condensation), as viewed by light or electron microscopy; and/or DNA cleavage into nucleosome-sized fragments, as determined by centrifuged sedimentation assays. Cell death occurs when the membrane integrity of the cell is lost (e.g., membrane blebbing) with engulfment of intact cell fragments ("apoptotic bodies") by phagocytic cells.

As used herein the term "biological activity", when used in the context of an analog, derivative, fragment, homolog or variant of a peptide or polypeptides means that the analog, derivative, fragment, homolog or variant retains at least one activity of the peptide or polypeptide including, but not limited to, the ability to be bound by a specific antibody, and when used in the context of WRN includes, but is not limited to, helicase activity, 3' to 5' exonuclease activity, and the ability to interact with the MRN complex; and, when used in the context of an analog, derivative, fragment, homolog or variant of an oligonucleotide means that the analog, derivative, fragment, homolog or variant hybridizes to the oligonucleotide under stringent hybridization conditions, as described by Maniatis et al., in Molecular Cloning (A Laboratory Manual), Cold Spring Harbor Laboratory, 1982, the contents of which are incorporated herein by reference.

As used herein, the term "cancer treatment" means any treatment for cancer known in the art including, but not limited to, chemotherapy and radiation therapy.

As used herein, the term "combination with" when used to describe administration of a modulator and an additional treatment means that the modulator may be administered prior to, together with, or after the additional treatment, or a combination thereof.

As used herein, the term "derivative", when used in the context of a peptide or polypeptide, means a peptide or polypeptide different other than in primary structure (amino acids and amino acid analogs), and preferably retains at least one biological activity; and, when used in the context of an oligonucleotide, means an oligonucleotide different other than in the nucleotide sequence, and preferably retains at least one biological activity. By way of illustration, derivatives of a peptide or polypeptide may differ by being glycosylated, one form of post-translational modification. For example, peptides or polypeptides may exhibit glycosylation patterns due to expression in heterologous systems. If at least one biological activity is retained, then these peptides or polypeptides are derivatives according to the invention. Other derivatives include, but are not limited to, fusion peptides or fusion polypeptides having a covalently modified N- or C-terminus, PEGylated peptides or polypeptides, peptides or polypeptides associated with lipid moieties, alkylated peptides or polypeptides, peptides or polypeptides linked via an amino acid side-chain functional group to other peptides, polypeptides or chemicals, and additional modifications as would be understood in the art.

As used herein, the term "fragment", when used in the context of a peptide or polypeptide, means any peptide or polypeptide fragment, preferably from about 5 to about 300 amino acids in length, more preferably from about 8 to about 50 amino acids in length, and preferably retains at least one biological activity; and, when used in the context of an oligonucleotide, means any oligonucleotide fragment, preferably from about 2 to about 250 nucleotides, more preferably from about 2 to about 20 nucleotides in length, and preferably retains at least one biological activity. Representative examples of peptide or polypeptide fragments are 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acids in length. Representative examples of oligonucleotide fragments are 2, 3, 4, 5, 6, 7, 8, 9, 10, 11, 12, 13, 14, 15, 16, 17, 18, 19 or 20 nucleotides in length.

As used herein, the term "homolog", when used in the context of a peptide or polypeptide, means a peptide or polypeptide sharing a common evolutionary ancestor or having at least 50% identity thereto, and preferably retains at least one biological activity; and, when used in the context of an oligonucleotide, means an oligonucleotide sharing a common evolutionary ancestor or having at least 50% identity thereto, and preferably retains at least one biological activity.

As used herein, the term "inhibit' when referring to the activity of a protein, means preventing, suppressing, repressing, or eliminating the activity of the enzyme.

As used herein, the term "treat" or "treating" when referring to protection of a mammal from a condition, means preventing, suppressing, repressing, or eliminating the condition. Preventing the condition involves administering a composition of the present invention to a mammal prior to onset of the condition. Suppressing the condition involves administering a composition of the present invention to a mammal after induction of the condition but before its clinical appearance. Repressing the condition involves administering a composition of the present invention to a mammal after clinical appearance of the condition such that the condition is reduced or prevented from worsening. Elimination of the condition involves administering a composition of the present invention to a mammal after clinical appearance of the condition such that the mammal no longer suffers from the condition.

As used herein, the term variant", when used in the context of a peptide or polypeptide, means a peptide or polypeptide that differs in amino acid sequence by the insertion, deletion, or conservative substitution of amino acids, and preferably retains at least one biological activity; and, when used in the context of an oligonucleotide, means an oligonucleotide that differs in nucleotide sequence by the insertion, deletion, or substitution of nucleotides, and preferably retains at least one biological activity.

### 2. Modulators

### a. Modulator of WRN

The present invention relates to a modulator of WRN activity. The modulator may induce or increase WRN activity. The modulator may also inhibit or reduce WRN activity. The modulator may be an artificially synthesized compound or a naturally occurring compound. The modulator may be a low molecular weight compound, oligonucleotide, polypeptide or peptide, or a fragment, analog, homolog, variant or derivative thereof.

An oligonucleotide modulator may be an oligonucleotide with at least about 33% to about 100% or at least 50% to about 100% nucleotide sequence identity with (TTAGGG)ₙ, wherein n is from about 1 to about 20. As used herein, "(TTAGGG)ₙ", when used in the context of a comparison of nucleic sequence identity, refers to a reference nucleic acid. Sequence identity is calculated by performing an alignment of the oligonucleotide and the reference nucleic acid and dividing (a) the number of identical nucleotides in the alignment, by (b) the total number of base pairs of the oligonucleotide. For example, the oligonucleotide may be 11-bp with the sequence GTTAGGGTTAG which has >91% sequence identity with (TTAGGG)₂.

The oligonucleotide may be of a form including, but not limited to, single-stranded, double-stranded, or a combination thereof The oligonucleotide preferably comprises a single-stranded 3'-end of from about 2 to about 2000 nucleotides, more preferably from about 2 to about 200 nucleotides. The oligonucleotide may also be an EST. Also specifically contemplated is an analog, derivative, fragment, homolog or variant of the oligonucleotide.

As shown in the Examples, certain oligonucleotides of the present invention caused the inhibition of proliferation and induction of apoptosis in cells, whereas other oligonucleotides of the present invention cause the inhibition of growth arrest and inhibition of apoptosis. The difference in the activity of the oligonucleotides was dependent on the number of 3' hydrolyzable internucleotide linkages. By varying the number of 3' hydrolyzable internucleotide bonds, the effect of the oligonucleotides was varied.

Not being bound by theory, we believe that the oligonucleotides are recognized by the WRN and serve as a substrate for the 3'-exonuclease WRN. The corollary is that substrate oligonucleotides that comprise 3'-nonhydrolyzable internucleotide bonds act as antagonists or inhibitors of WRN. Other factors determining the level of WRN activity include, but are not limited to, the total concentration of 3'-hydrolyzable internucleotide bonds, base sequence and G content.

An internucleotide bond is considered hydrolyzable for purposes of the present invention if (i) it is a phosphodiester linkage or an analog thereof that is hydrolyzable by WRN under physiological conditions, and (ii) all internucleotide bonds 3' thereto are also hydrolyzable. An internucleotide bond is considered nonhydrolyzable for purposes of the present invention if it is not hydrolyzable by WRN under physiological conditions, regardless of the number of hydrolyzable internucleotide linkages that are 3' thereto. Representative examples of nonhydrolyzable internucleotide linkages include, but are not limited to, phosphorothioate linkages and peptide nucleic acid linkages (PNA).

In one embodiment of the invention, the oligonucleotide comprises hydrolyzable internucleotide bonds. The oligonucleotide may comprise from about 1 to about 200 hydrolyzable internucleotide bonds. The oligonucleotide may also comprise nonhydrolyzable internucleotide bonds. The oligonucleotide may comprise from about 0 to about 199 nonhydrolyzable internucleotide bonds.

In another embodiment, the oligonucleotide comprises nonhydrolyzable bonds. The oligonucleotide may comprise from about 1 to about 200 nonhydrolyzable internucleotide bonds. The oligonucleotide may also comprise hydrolyzable internucleotide bonds. The oligonucleotide comprise from about 0 to about 5 hydrolyzable internucleotide bonds. Preferred oligonucleotides are T-oligos described herein and as described in co-pending U.S. Patent Application No. 10/122,630, filed April 12, 2002, which is incorporated herein by reference.

### b. Modulator of the DNA Damage Pathway

The present invention also relates to a modulator of the DNA damage pathway. The modulator may induce the DNA damage pathway. The modulator may also inhibit the DNA damage pathway. The modulator may be an artificially synthesized compound or a naturally occurring compound. The modulator may be a low molecular weight compound, polypeptide or peptide, or a fragment, analog, homolog, variant or derivative thereof.

### 3. Composition

The present invention also relates to a composition comprising a modulator as described above. The composition may comprise an activator of WRN. The composition may also comprise an inhibitor of WRN. The composition may also comprise more than one modulator of the present invention. The composition may also comprise one or more modulators together with an additional therapeutic.

In one embodiment of the present invention, the composition comprises an oligonucleotide of the present invention. The oligononucleotide may comprise hydrolyzable internucleotide bonds or nonhydrolyzable internucleotide bonds, or a combination thereof. In a preferred embodiment, the oligonucleotide is an activator of WRN. In another preferred embodiment, the oligonucleotide is an inhibitor of WRN. As discussed above, the activity of the oligonucleotide may be adjusted to induce or inhibit WRN based on the total concentration of hydrolyzable internucleotide bonds.

### a. Formulation

Compositions of the present invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate). Tablets may be coated according to methods well known in the art.

Compositions of the present invention may also be liquid formulations including, but not limited to, aqueous or oily suspensions, solutions, emulsions, syrups, and elixirs. The compositions may also be formulated as a dry product for constitution with water or other suitable vehicle before use. Such liquid preparations may contain additives including, but not limited to, suspending agents, emulsifying agents, nonaqueous vehicles and preservatives. Suspending agent include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid.

Compositions of the present invention may also be formulated as suppositories, which may contain suppository bases including, but not limited to, cocoa butter or glycerides. Compositions of the present invention may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane. Compositions of the present invention may also be formulated transdermal formulations comprising aqueous or nonaqueous vehicles including, but not limited to, creams, ointments, lotions, pastes, medicated plaster, patch, or membrane.

Compositions of the present invention may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of the present invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

Compositions of the present invention may also be formulated as a liposome preparation. The liposome preparation can comprise liposomes which penetrate the cells of interest or the stratum corneum, and fuse with the cell membrane, resulting in delivery of the contents of the liposome into the cell. For example, liposomes such as those described in U.S. Patent No. 5,077,211 of Yarosh, U.S. Patent No. 4,621,023 of Redziniak et al. or U.S. Patent No. 4,508,703 of Redziniak et al. can be used. The compositions of the invention intended to target skin conditions can be administered before, during, or after exposure of the skin of the mammal to UV or agents causing oxidative damage. Other suitable formulations can employ niosomes. Niosomes are lipid vesicles similar to liposomes, with membranes consisting largely of non-ionic lipids, some forms of which are effective for transporting compounds across the stratum corneum.

### 4. Methods of Treatment

### a. Activator of WRN

The modulators of the present invention that induce or increase the activity of WRN may be used alone or in combination with other treatments to treat conditions associated with failure of growth arrest, apoptosis or proliferative senescence. Representative examples of such conditions include, but are not limited to, hyperproliferative diseases, such as cancer and the benign growth of cells beyond a normal range as, for example, keratinocytes in psoriasis or fibroblast hypertrophic scars and keloids, or certain subsets of lymphocytes in the case of certain autoimmune disorders. The forms of cancer to be treated by these methods are manifested in various forms and arising in various cell types and organs of the body, for example, cervical cancer, lymphoma, osteosarcoma, melanoma and other cancers arising in the skin, and leukemia. Also among the types of cancer cells to which the therapies are directed are breast, lung, liver, prostate, pancreatic, ovarian, bladder, uterine, colon, brain, esophagus, stomach, and thyroid. The modulators may also be used to inhibit promote tanning, to promote cellular differentiation and for immunosuppresion.

In one embodiment of the present invention, an oligonucleotide of the present invention comprising hydrolyzable internucleotide bonds is used to treat a condition associated with failure of growth arrest, apoptosis or proliferative senescence by administering the oligonucleotide to a patient in need of such treatment. The oligononucleotide may also comprise nonhydrolyzable internucleotide bonds. As discussed above, the activity of the oligonucleotide may be adjusted to induce growth arrest or apoptosis based on the total concentration of hydrolyzable internucleotide bonds. The oligonucleotide may be administered in combination with modulators of the present invention or other treatments.

In a preferred embodiment, the oligonucleotide is used to treat a cancer selected from the group consisting of cervical, lymphoma, osteosarcoma, melanoma, skin, leukemia, breast, lung, liver, prostate, pancreatic, ovarian, bladder, uterine, colon, brain, esophagus, stomach, and thyroid.

T-oligos are capable of blocking induction or elicitation of allergic contact hypersensitivity as effectively as UV irradiation in a mouse model, through upregulation of TNF-α and IL10, known mediators of immunosuppression. A topical or systemic activator of WRN may, therefore, replace steroid therapy, for example, in treatment of lymphocyte-mediated skin diseases, such as psoriasis or eczema as well as lymphocyte-mediated systemic diseases such as rheumatoid arthritis, multiple sclerosis, lupus erythematosis, and many other diseases.

### b. Inhibitor of WRN

The modulators of the present invention that inhibit or decrease the activity of WRN may be used alone or in combination with other treatments to treat conditions associated with growth arrest, apoptosis or proliferative senescence. Representative examples of such conditions include, but are not limited to, exposure to UV radiation and side effects of cancer treatments on normal tissues, such as chemotherapy and radiation therapy, or promoting inhibiting the tanning response in sun exposed normal skin. The modulators may also be used to inhibit cellular differentiation.

In another embodiment, an oligonucleotide of the present invention comprising nonhydrolyzable internucleotide bonds is used to treat a condition associated with growth arrest or apoptosis by administering the oligonucleotide to a patient in need of such treatment. The oligononucleotide may also comprise hydrolyzable internucleotide bonds. As discussed above, the activity of the oligonucleotide may be adjusted to inhibit growth arrest or inhibit apoptosis based on the total concentration of hydrolyzable internucleotide bonds. The oligonucleotide may be administered in combination with modulators of the present invention or other treatments.

In a preferred embodiment, the oligonucleotide is used to treat a condition selected from the group consisting of exposure to UV radiation and side effects of cancer treatments, such as chemotherapy and radiation therapy.

### c. Administration

Compositions of the present invention may be administered in any manner including, but not limited to, orally, parenterally, sublingually, transdermally, rectally, transmucosally, topically, via inhalation, via buccal administration, or combinations thereof. Parenteral administration includes, but is not limited to, intravenous, intraarterial, intraperitoneal, subcutaneous, intramuscular, intrathecal, and intraarticular.

### d. Dosage

A therapeutically effective amount of the composition required for use in therapy varies with the nature of the condition being treated, the length of time that activity is desired, and the age and the condition of the patient, and is ultimately determined by the attendant physician. In general, however, doses employed for adult human treatment typically are in the range of 0.001 1 mg/kg to about 200 mg/kg per day. The dose may be about 1 µg/kg to about 100 µg/kg per day. The desired dose may be conveniently administered in a single dose, or as multiple doses administered at appropriate intervals, for example as two, three, four or more subdoses per day. Multiple doses often are desired, or required.

The dosage of a modulator may be at any dosage including, but not limited to, about 1 µg/kg, 25 µg/kg, 50 µg/kg, 75 µg/kg, 100 µg/kg, 125 µg/kg, 150 µg/kg, 175 µg/kg, 200 ug/kg, 225 µg/kg, 250 µg/kg, 275 ug/kg, 300 ug/kg, 325 µg/kg, 350 µg/kg, 375 µg/kg, 400 µg/kg, 425 µg/kg, 450 µg/kg, 475 µg/kg, 500 µg/kg, 525 µg/kg, 550 ug/kg, 575 ug/kg, 600 µg/kg, 625 µg/kg, 650 µg/kg, 675 µg/kg, 700 µg/kg, 725 µg/kg, 750 µg/kg, 775 µg/kg, 800 ug/kg, 825 µg/kg, 850 µg/kg, 875 µg/kg, 900 µg/kg, 925 µg/kg, 950 µg/kg, 975 µg/kg or 1 mg/kg.

### 5. Screening Methods

The present invention also relates to screening methods of identifying modulators of WRN activity. Furthermore, the present invention relates to screening methods of identifying modulators of the DNA damage pathway. The screening methods may be performed in a variety of formats including, but not limited to, *in vitro,* cell-based, genetic and *in vivo* assays.

Modulators of WRN may be identified by screening for substances that specifically bind to WRN. Specific binding substances may be identified *in vitro* by one of ordinary skill in the art using a number of standard techniques including, but not limited to, immunoprecipitation and affinity chromatography. Specific binding substances may also be identified using genetic screens by one of ordinary skill in the art using a number of standard techniques including, but not limited to, yeast two-hybrid and phage display. Specific binding substances may also be identified using high throughput screening methods including, but not limited to, attaching WRN to a solid substrate such as a chip (e.g., glass, plastic or silicon).

Modulators of WRN may also be identified by screening *in vitro* for substances that modulate the activity of WRN. Modulators may be identified by contacting WRN with a suspected modulator and determining whether the suspected modulator alters the activity of WRN. The activity of WRN may be determined by measuring the hydrolysis of a nucleic acid substrate of WRN. Hydrolysis of a nucleic acid substrate may be measured by methods including, but not limited to, measuring UV absorbance and, preferably, gel analysis of labeled oligos or recovery of non-precipitatable nucleotide bases.

A modulator of WRN may be identified by screening for substances that modulate the activity of WRN in cell-based assays. A modulator of the DNA damage pathway may similarly be identified. Modulators may be identified by contacting cells with a suspected modulator and determining whether the suspected modulator alters the level of apoptosis, senescence, or phosphorylation of p53, p95, ATM, H2AX, induction of S phase arrest or induction of apoptosis. The candidate modulator may be a substance that specifically binds to WRN, as discussed above. Modulation of apoptosis may be measured by methods including, but not limited to, measuring the size of the sub-G_{0/}G₁ peak in FACS analysis, TUNEL assay, DNA ladder assay, annexin assay, or ELISA assay. Modulation of senescence may be determined by measuring senescence-associated β-galactosidase activity or failure to increase cell yields or to phosphorylate pRb or to incorporate ³H-thymidine after mitogenic stimulation. Modulation of p53 activity may be determined by measuring phosphorylation of p53 at serine 15 or serine 37 by gel shift assay by p53 promoter driven CAT or luciferase construct read-out, or by induction of a p53-regulated gene product such as p21. Modulation of p95 activity may be determined by measuring phosphorylation of p95 at serine 343 by shift in the p95 band in a western blot analysis, or by FACS analysis to detect an S phase arrest. A modulator of WRN may also be identified by screening for substances that modulate in *vivo* tumorigenecity.

Any cells may be used with cell-based assays. Preferably, cells for use with the present invention include mammalian cells, more preferably human and non-human primate cells. Representative examples of suitable cells include, but are not limited to, primary (normal) human dermal fibroblasts, epidermal keratinocytes, melanocytes, and corresponding immortalized or transformed cell lines; and primary, immortalized or transformed murine cells lines. The amount of protein phosphorylation may be measured using techniques standard in the art including, but not limited to, colorimetery, luminometery, fluorimetery, and western blotting.

The conditions under which a suspected modulator is added to a cell, such as by mixing, are conditions in which the cell can undergo apoptosis or signaling if essentially no other regulatory compounds are present that would interfere with apoptosis or signaling. Effective conditions include, but are not limited to, appropriate medium, temperature, pH and oxygen conditions that permit cell growth. An appropriate medium is typically a solid or liquid medium comprising growth factors and assimilable carbon, nitrogen and phosphate sources, as well as appropriate salts, minerals, metals and other nutrients, such as vitamins, and includes an effective medium in which the cell can be cultured such that the cell can exhibit apoptosis or signaling. For example, for a mammalian cell, the media may comprise Dulbecco's modified Eagle's medium containing 10% fetal calf serum.

Cells may be cultured in a variety of containers including, but not limited to tissue culture flasks, test tubes, microtiter dishes, and petri plates. Culturing is carried out at a temperature, pH and carbon dioxide content appropriate for the cell. Such culturing conditions are also within the skill in the art.

Methods for adding a suspected modulator to the cell include electroporation, microinjection, cellular expression (i.e., using an expression system including naked nucleic acid molecules, recombinant virus, retrovirus expression vectors and adenovirus expression), adding the agent to the medium, use of ion pairing agents and use of detergents for cell permeabilization.

Candidate modulators may be naturally-occurring molecules, such as carbohydrates, monosaccharides, oligosaccharides, polysaccharides, amino acids, peptides, oligopeptides, polypeptides, proteins, nucleosides, nucleotides, oligonucleotides, polynucleotides, including DNA and DNA fragments, RNA and RNA fragments and the like, lipids, retinoids, steroids, glycopeptides, glycoproteins, proteoglycans and the like; or analogs or derivatives of naturally-occurring molecules, such peptidomimetics and the like; and non-naturally occurring molecules such as "small molecule" organic compounds. The term "small molecule organic compound" refers to organic compounds generally having a molecular weight less than about 1000, preferably less than about 500.

Candidate modulators may be present within a library (i.e., a collection of compounds), which may be prepared or obtained by any means including, but not limited to, combinatorial chemistry techniques, fermentation methods, plant and cellular extraction procedures and the like. Methods for making combinatorial libraries are well-known in the art. See, for example, E. R. Felder, Chimia 1994, 48, 512-541; Gallop et al., J. Med. Chem. 1994, 37, 1233-1251; R. A. Houghten, Trends Genet. 1993, 9, 235-239; Houghten et al., Nature 1991, 354, 84-86; Lam et al., Nature 1991, 354, 82-84; Carell et al., Chem. Biol. 1995, 3, 171-183; Madden et al., Perspectives in Drug Discovery and Design 2, 269-282; Cwirla et al., Biochemistry 1990, 87, 6378-6382; Brenner et al., Proc. Natl. Acad. Sci. USA 1992, 89, 5381-5383; Gordon et al., J. Med. Chem. 1994, 37, 1385-1401; Lebl et al., Biopolymers 1995, 37 177-198; and references cited therein.

The present invention has multiple aspects, illustrated by the following non-limiting examples.

### EXAMPLES

### Example 1

### Oligonucleotides can induce apoptosis

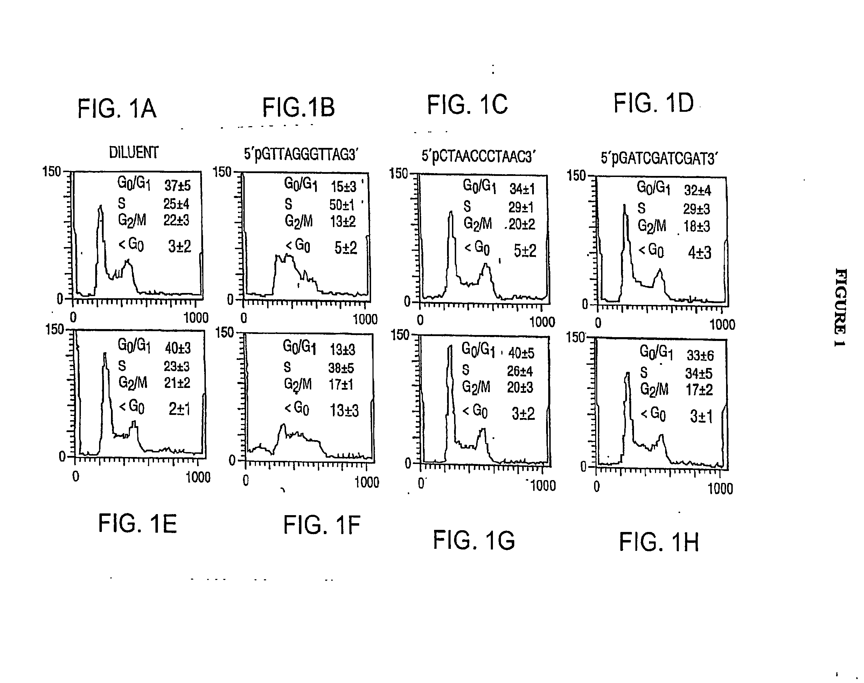

Oligonucleotides homologous to the telomere overhang repeat sequence (TTAGGG; SEQ ID NO: 1), sequence (11mer-1: pGTTAGGGTTAG; SEQ ID NO: 2), complementary to this sequence (11mer-2: pCTAACCCTAAC; SEQ ID NO: 3) and unrelated to the telomere sequence (11mer-3: pGATCGATCGAT; SEQ ID NO: 4) were added to cultures of Jurkat cells, a line of human T cells reported to undergo apoptosis in response to telomere disruption. Within 48 hours, 50% of the cells treated with 40 µM of SEQ ID NO: 2 had accumulated in the S phase, compared to 25-30% for control cells (p<0.0003, non-paired t-test; see Figures 1A-1D), and by 72 hours, 13% of these cells were apoptotic as determined by a sub-G₀/G₁ DNA content, compared to 2-3% of controls (p<0.007, non-paired t-test; see Figures 1E-1H). At 96 hours, 20±3% of the 11 mer-1 treated cells were apoptotic compared with 3-5% of controls (p<0.0001, non-paired t-test). To exclude preferential uptake of the 11mer-1 as an explanation of its singular effects, Jurkat cells were treated with oligonucleotides labeled on the 3' end with fluorescein phosphoramidite, then subjected to confocal microscopy and FACS analysis. The fluorescence intensity of the cells was the same after all treatments at 4 hours and 24 hours. Western analysis showed an increase in p53 by 24 hours after addition of 11mer-1, but not 11mer-2 or 1 lmer-3, with a concomitant increase in the level of the E2F1 transcription factor, which is known to cooperate with p53 in induction of apoptosis and to induce a senescent phenotype in human fibroblasts in a p53-dependent manner as well as to regulate an S phase checkpoint.

### Example 2

### Phosphorothioate Version of the Telomere Overhang Homolog 11mer-1 Does Not Induce Apoptosis

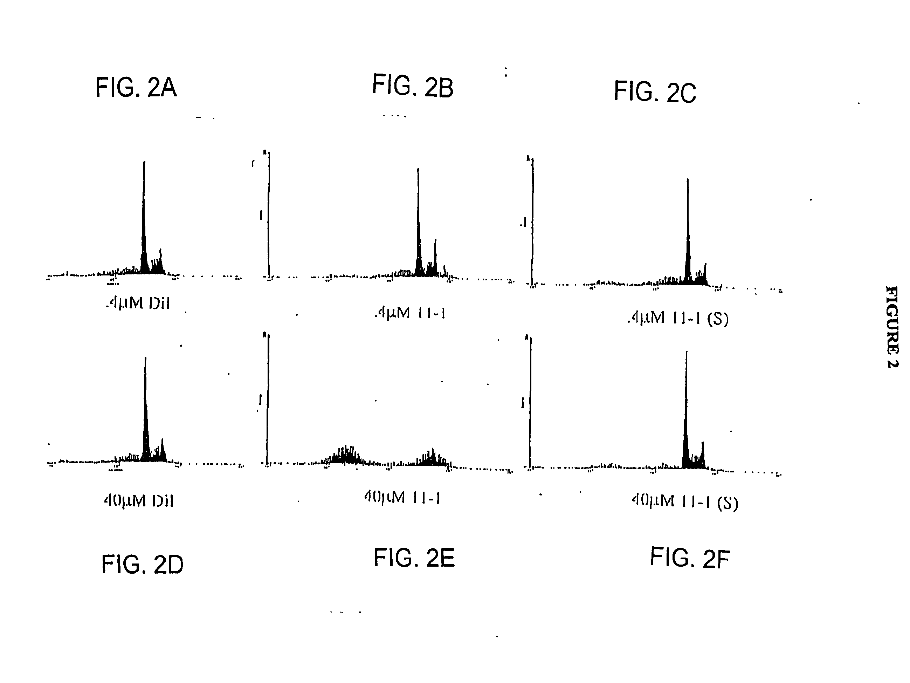

Cultures of Jurkat human T cells were treated with either diluent, 11mer-1 (SEQ ID NO: 1) or the phosphorothioate 11mer- (11mer-1-S) for 96 hours, then collected and processed for FACS analysis. Two concentrations of the oligonucleotides were tested, 0.4 µM (Figures 2A-2C) and 40 µM (Figures 2D-2F). At 0.4 µM, neither of the oligonucleotides affected the expected exponentially growing cell cycle profile of the Jurkat cells. At 40 µM, the 11-mer-2 induced extensive apoptosis, indicated by a sub-G₀/G₁ peak, while the 11mer-1-S had no effect.

### Example 3

### Phosphorothioate Version of 11mer-1 Blocks Induction of S-Phase Arrest by the Phosphate Backbone 11mer-1

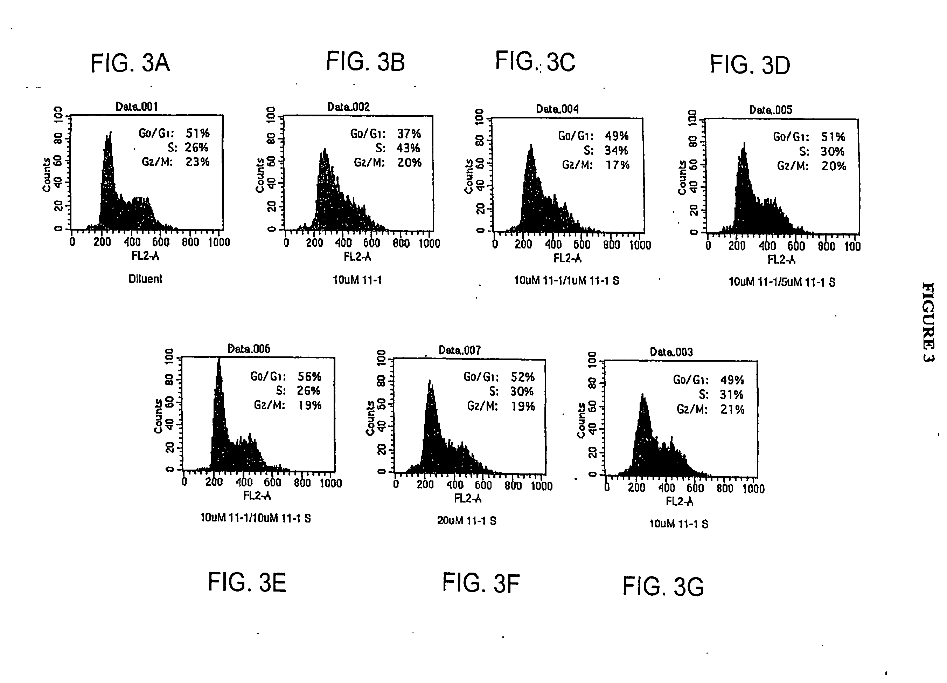

Cultures of a keratinocyte cell line (SSC12F, 100,000 cells/38 cm²) were treated for 48 hours with only the 11mer-1 (SEQ ID NO: 2) or with the 11mer-1 in the presence of increasing concentrations of the 11mer-1-S. As shown previously in Example 1, the 11mer-1 induced an S-phase arrest as demonstrated by FACS (Becton-Dickinson FacScan). Forty-three percent of the cells were in the S phase, compared to 26% of the control, diluent-treated cells. However, when increasing concentrations of the phosphorothioate 11mer-1 were also added to these cultures, fewer cells became arrested (Figures 3A-3G). Complete inhibition of this arrest was seen with a ratio of 11mer-1: 11mer-1-S of 2:1. The 11 lmer-1-S by itself did not induce the S-phase arrest.

### Example 4

### Phosphorothioate Forms of the Telomere Oligonucleotides Reduce Constitutive and UV-Induced Pigmentation and Do Not Stimulate Melanogenesis

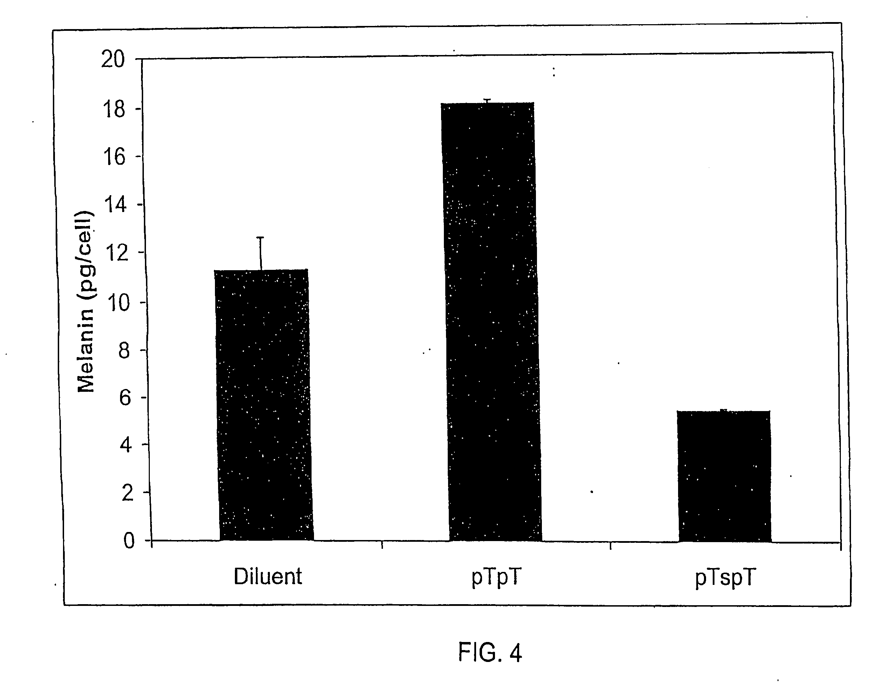

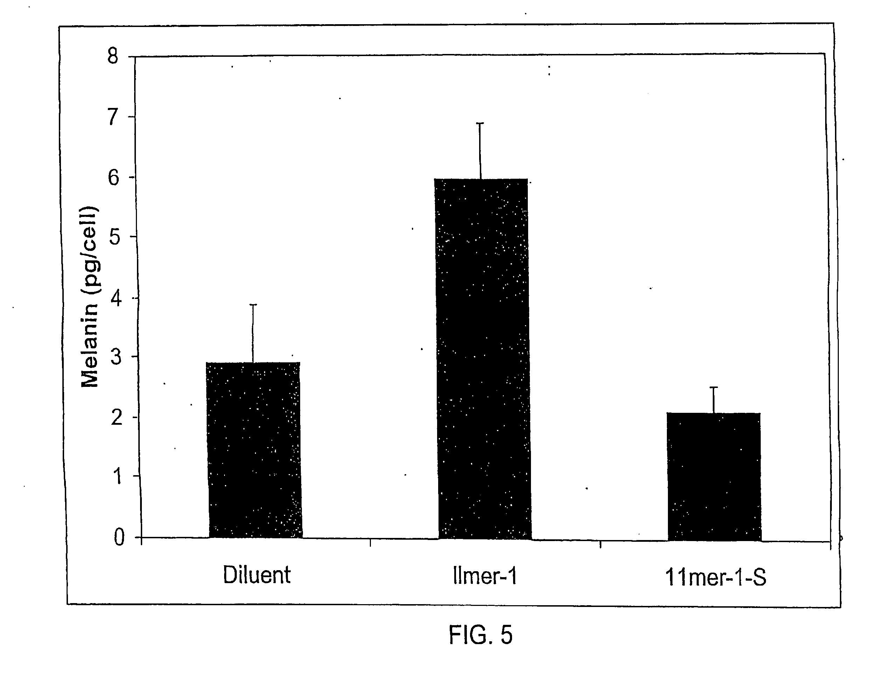

Cultures of S91 mouse melanoma cells (100,000 cells/38 cm²) were treated with 100 µM pTpT or phosphorothioate pTpT (pTspT) (Figure 4) or 40 uM 11mer-1 or the phosphorothioate 11mer-1(11mer-1-S) (Figure 5) for 6 days and were then collected, counted and assayed for melanin content. While the pTpT and 11mer-1 (Figure 4 and Figure 5, respectively) stimulated melanogenesis in these cells, pTspT and 11mer-1-S did not (Figure 4 and Figure 5, respectively). Furthermore, both pTspT (Figure 4) and 11mer-1-S (Figure 5) reduced the constitutive pigmentation in these cells, suggesting that chronic exposure of this sequence during telomere repair/replication may provide a constant, low level signal for melanogenesis and this signal is blocked by pTspT and 1 lmer-1-S.

### Example 5

### Phosphorothioate pTspT Inhibits UV-Induced Melanogenesis

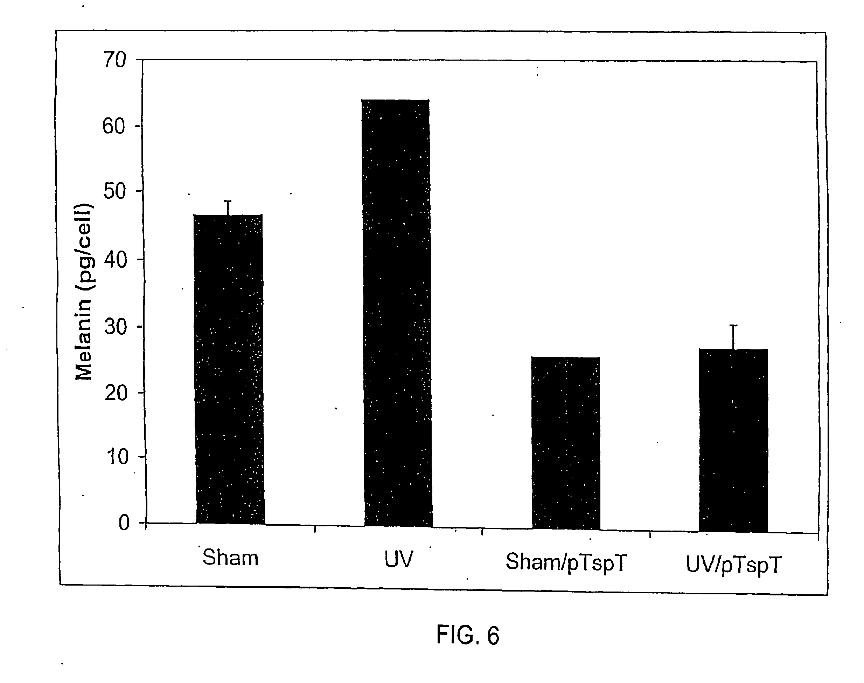

Duplicate cultures of S91 cells (100,000 cells/39 cm²) were either sham-irradiated or irradiated with 5 mJ/cm² solar-simulated light from a 1 kW xenon arc solar-simulator (XMN 1000-21, Optical Radiation, Azuza, CA) metered at 285 5 nm using a research radiometer (model IL1700A, International Light, Newburyport, MA). Two sham-irradiated plates were then supplemented with 100 µM pTspT and two irradiated cultures were similarly treated with pTspT. After one week, cells were collected, counted and analyzed for melanin content by dissolving the cell pellets in 1 N NaOH and measuring the optical density at 475 nm. UV irradiation resulted in a doubling of melanin content in these cells. However, this response was blocked by the addition of pTspT (Figure 6). In addition, the constitutive pigmentation of these cells was reduced by the pTspT in the sham-irradiated cultures, similar to the data presented in Figures 4 and 5.

### Example 6

### Hydrolysis of the T-oligo is Necessary for Activity

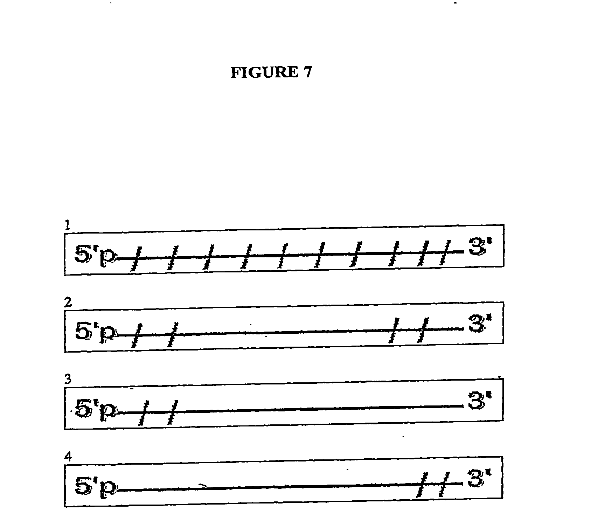

Oligonucleotides based on SEQ ID NO: 2 were synthesized. Oligonucleotide 1 was synthesized entirely with a phosphorothioate backbone. Oligonucleotide 2 had two phosphorothioate linkages on each end, with the other linkages in the middle being phosphodiester linkages. Oligonucleotide 3 had two phosphorothioate linkages on the 5' end (5' end blocked), with the rest of the linkages being phosphodiester linkages. Oligonucleotide 4 had two phosphorothioate linkages on the 3' end (3' end blocked), with the rest of the linkages being phosphodiester linkages. See Figure 7.

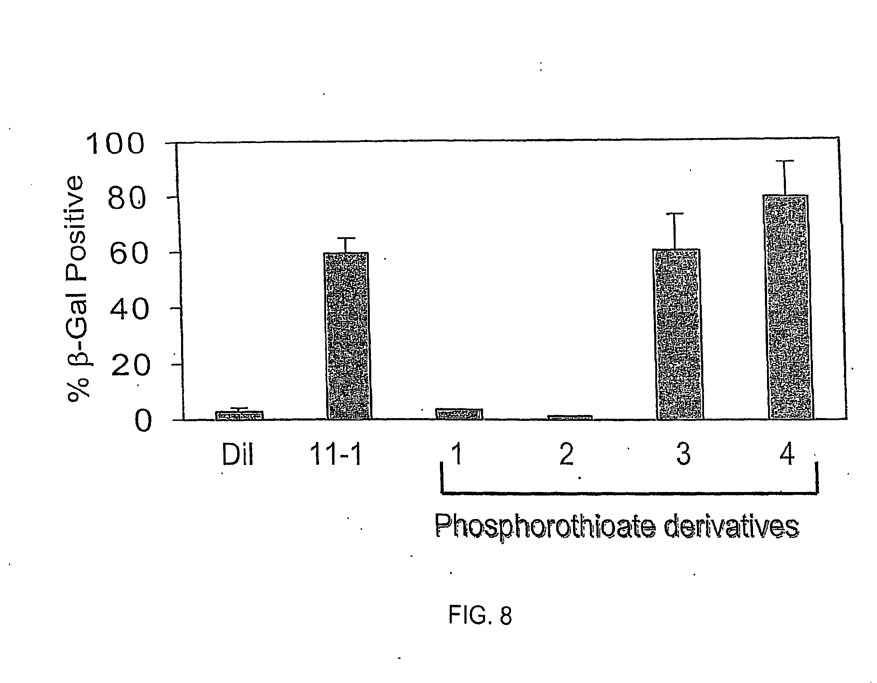

These oligonucleotides were added to cultures of normal neonatal fibroblasts. After 48 hours, cells were collected to be analyzed for p53 serine 15 phosphorylation and p95/Nbsl phosphorylation by western blot. Other cultures were left in the presence of the oligonucleotides for one week and then the cells were stained for senescence-associated β-galactosidase activity (SA-β-Gal) β-galactosidase positive cells were scored and presented as a percent of total cells (Figure 8).

Oligonucleotides with a nuclease-accessible 3' terminus are the most effective at stimulating "early" responses such as p53 and p95/Nbsl phosphorylation. However, oligonucleotides with a nuclease-accessible 5' terminus can also induce the senescent phenotype after one week, but not the phosphorylation reactions at 48 hours, suggesting that 3' to 5' nuclease susceptibility is preferable for activity in inducing senescence.

### Example 7

### Downregulating WRN Protein Levels Blocks Response of T-Oligos

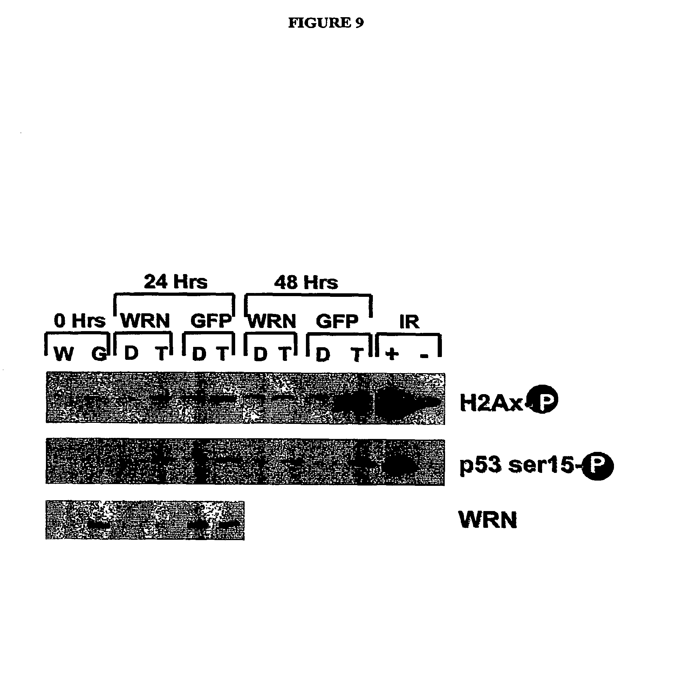

Normal neonatal fibroblasts were treated on 2 consecutive days with 50 pmol of WRN siRNA or 50 pmol of siRNA directed against the control green fluorescent protein (GFP). One day after the second siRNA treatment, representative cultures were collected for western blot analysis to assess the effectiveness of the WRN siRNA in eliminating the protein. Also at this time, duplicate cultures of WRN siRNA-treated or GFP siRNA-treated cultures were given either 40 µM T-oligo (11mer-1; SEQ ID NO: 2) or an equal amount of diluent. Cells from all conditions were collected one or two days after the addition of T-oligo or diluent and were analyzed by western blot for p53 phosphorylation on serine 15 and phosphorylation of histone H2AX, both well-documented DNA damage responses (Lambert et al., 1998, J Biol Chem 273, 33048-53; Burma et al., 2001, J Biol Chem 276, 42462-7). Also as controls, cell lysates from cells sham-irradiated or irradiated with 10 Gy ionizing radiation (IR) were included. The blot was probed with antibodies specific for WRN, p53 phosphoserine 15 and phospho-H2AX. The data presented in Figure 9 demonstrate that "knock-down" of WRN dramatically reduced the level of WRN on day 0 (the day of T-oligo treatment) and the ability of T-oligo to induce the phosphorylation of p53 and H2AX.

### Example 8

### DNA-PK mediates effects of T-oligos

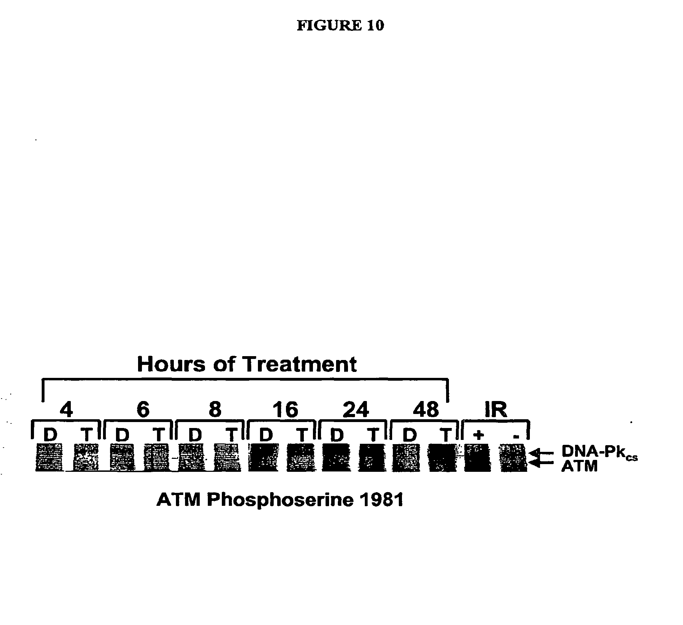

Normal newborn fibroblasts were treated with 40µM T-oligo or an equal volume of diluent for 4, 6, 8, 16, 24 or 48 hours then collected and analyzed by Western blot using an antibody against ATM phosphoserine 1981. Figure 10 shows that T-oligo treatment in fibroblasts leads to phosphorylation of a protein with an apparent molecular mass of 450 kDa that migrated above the marker for ATM (370 kDa). The phosphorylated protein was shown in subsequent experiments to co-migrate with the catalytic subunit of ATM-related DNA-dependent protein kinase (DNA-PK_{cs}), which would reasonably be expected to bind an antibody raised against the highly homologous ATM protein.

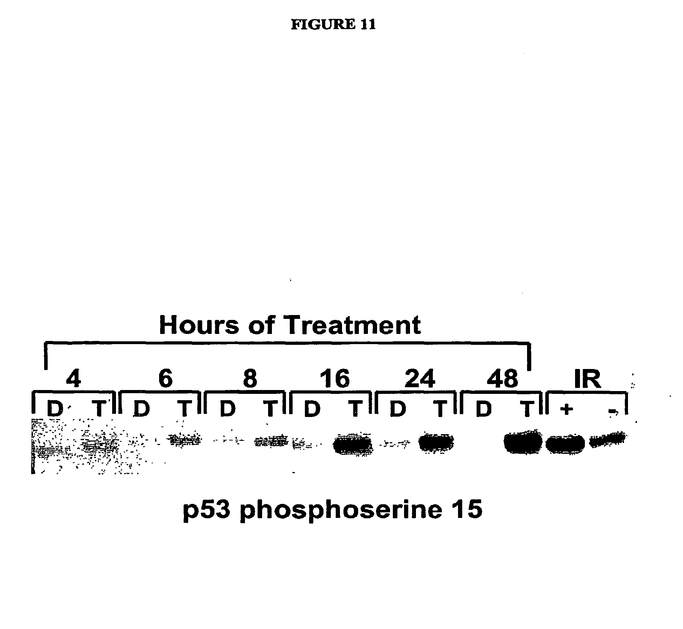

Because DNA-PK_{cs} autophosphorylates when activated (Ding et al., 2003, Mol Cell Biol 23, 5836-48, as suggested to happen after T-oligo treatment (Figure 10), and also associates with WRN through the heterodimer Ku protein (part of the DNA-PK complex)⁵, fibroblasts were analyzed for p53 phosphorylation on serine 37, a site shown to be phosphorylated by DNA-PK (Lees et al., 1992, Mol Cell Biol 12, 5041-9). Newborn fibroblasts were treated as described above and similarly analyzed using an antibody against p53 phosphorylated on serine 37. Western analysis showed that p53 serine 37 is phosphorylated in response to T-oligo treatment (Figure 11).

## Claims

1. A composition comprising an activator or inhibitor of WRN for use as a medicament

2. A composition comprising an activator of WRN for use as a medicament according to claim 1, wherein the use comprises the treatment of cancer the induction of apoptosis, inducing cellular senescence, promoting tanning, promoting cellular differentiation or promoting immunosuppression.

3. A composition according to claim 2, wherein the use comprises the treatment of cancer.

4. A composition according to claim 2, wherein the use comprises inducing apoptosis.

5. A composition according to claim 2, wherein the use comprises the treatment of inducing cellular senescence.

6. A composition according to claim 2, wherein the use comprises the treatment of promoting tanning.

7. A composition according to claim 2, wherein the use comprises the treatment of promoting cellular differentiation.

8. A composition according to claim 2, wherein the use comprises the treatment of promoting immunosuppression.

9. A composition comprising an inhibitor of WRN for use as a medicament according to claim 1, wherein the use comprises inhibition of apoptosis, inhibiting cellular senescence, promotion of growth, inhibiting tanning, inhibiting cellular differentiation or reducing cancer treatment side effects.

10. A composition according to claim 9, wherein the use comprises inhibiting apoptosis.

11. A composition according to claim 9, wherein the use comprises inhibiting cellular senescence.

12. A composition according to claim 9, wherein the use comprises promoting growth.

13. A composition according to claim 9, wherein the use comprises inhibiting tanning.

14. A composition according to claim 9, wherein the use comprises inhibiting cellular differentiation.

15. A composition according to claim 9, wherein the use comprises reducing cancer treatment side effects.

16. The composition of claim 15 wherein the composition is given in combination with chemotherapy or ionizing radiation.

17. The method of any one of claims 1-16 wherein the activator or inhibitor is an oligonucleotide activator or inhibitor of WRN with at least 33% nucleotide sequence identity with (TTAGGG)ₙ and at least the first x 3'-nucleotide linkages are hydrolyzable by a 3' to 5' nuclease, wherein n=1 to 20, and wherein x is from about 0 to about 10.

18. A composition comprising an oligonucleotide with at least 33% nucleotide sequence identity with (TTAGGG)ₙ wherein at least the first x 3'-nucleotide linkages are hydrolyzable by a 3' to 5' nuclease, wherein n=1 to 20, and wherein x is from about 0 to about 10.

19. The composition of claim 26 wherein the 3' to 5' nuclease is WRN.
